# EUROPEAN PATENT SPECIFICATION

(11) **EP 3 494 838 B1**
(45) Date of publication and mention of the grant of the patent: **15.04.2020**
(21) Application number: 17838859.1
(22) Date of filing: 08.08.2017
(51) Int. Cl.: A47C 1/08, A47C 3/30, A61B 90/60, A47C 1/06

(54) **NEW ERGONOMIC WORKSTATION WITH ASSISTED MOVEMENT FOR HAIRDRESSERS**
NEUER ERGONOMISCHER ARBEITSPLATZ MIT UNTERSTÜTZTER BEWEGUNG FÜR FRISEURE
NOUVEAU POSTE DE TRAVAIL ERGONOMIQUE AVEC UNE ASSISTANCE AU MOUVEMENT POUR DES COIFFEURS

(30) Priority: 10.08.2016 ES 201631091
(43) Date of publication of application: 12.06.2019
(73) Proprietor: Spin International, SLU, Andorra (AD)
(72) Inventor: PERALES BOTÍA, Javier, 46015 Valencia (ES)
(74) Representative: Chanza Jordan, Dionisio
(86) International application number: PCT/ES2017/070571
(87) International publication number: WO 2018/029390

(56) References cited:
- ES-U- 1 042 747
- FR-A1- 2 800 252
- JP-A- 2004 073 830
- US-A- 4 863 217
- US-A1- 2007 216 205

## Description

### Sector of the technique

This invention is intended for the professional furniture industry, more specifically, the industrial sector dedicated to the construction of workstations with assisted movement for hairdressers.

### Background

There are systems nowadays to facilitate the work of certain professionals such as hairdressers who, because of the nature of their work, have to spend long periods of time making movements and adopting postures which are potentially harmful for their musculoskeletal systems, a large number of individuals developing problems caused by these movements and postures. So patents are known such as the Japanese JP2006345879 with the title Work Assisting Device which describes a device suspended from the ceiling rigidly joined to a harness which comprises a chair in which the worker is placed, with conventional means such as a belt for attaching the worker firmly to the chair. The invention described in that patent requires a complex installation including the need for a structure for fixing to the ceiling and clearly restricts the work of the operator because of its complexity, so being different from the invention being put forward.

The utility model CN87204900U with the title Barber's working chair describes a barber's chair in which the latter sits on a support element for the buttocks and another for shin support, as well as a complicated system of rails enabling circular movement around the client's chair. These rails also hamper adequate cleaning of the chair and rails, because it is widely known that during a haircut, the floor becomes covered with hair. Again, this system is very complicated, at the same time being difficult to implement mechanically because of its high cost, and also gives the hairdresser little room for manoeuvre, so making it impractical. With this invention, the user of the chair must necessarily work sitting down, which hampers and even impedes his or her work, because certain techniques require the worker to be standing up to perform them.Therefore, inventions like this one are not known.

The proposed invention is related to the assisted movement for hairdressers on the basis of the gas piston located in the vertical part of the frame under the seat.No manual actions are required, because the elevation movement is agreed with the weight of the hairdressers to make easier the use of both free arms for the hairdressers works.

So, the piston tension applied by the gas is essential to an harmonius movement combined and simultaneous with the hairdressers weight.

Earlier patents as US2007/0216205 A1 describes a seat mount cylinder with a seat adjustment handle by manual means and FR 2800252 A1 describes a gaz cartridge is employed on a seat to facilitate an oscillating movement of the user.Those inventions are far from the lift system of a gas piston located in the vertical part of the frame under the seat, which is in its natural extended position when its valve is not operated, the latter being activated when the hairdresser makes a slight movement to tilt the backrest, use of his hands therefore being unnecessary, this action ordering activation of the piston and the consequent descent of the seat/backrest assembly through an activation system consisting of a set of levers.In short, all the vertical part of the frame under the seat is a gas piston lisf system, necessary to results of weights between 60- 120 kg of a human user, and an harmonius vertical movement.These results are imposible with manual and jumping adjustements as in US2007/0216205 A1 or a simple gaz cartridge to oscillating movements as in FR2800252 A1 height of the work plane and reachable areas in the horizontal plane, also enabling fluent turning movement around the client. In this way, this new workstation enables the hairdresser to work in optimum conditions of comfort. It has a saddle-like seat like those used in bicycles, so that, as well as being able to sit on it, the hairdresser can also stand without the seat preventing this position, and use it as additional support if he or she requires. The invention also has a backrest with armrests which can be height-adjusted by conventional means. The fact that a saddle-type seat is used gives the hairdresser's legs an amount of freedom not offered by a conventional chair.

Regulation of the hairdresser's height to adjust the work zone in relation to the client's head is carried out by the structure or frame of the invention having means such as a conventional lifting system such as a piston to raise or lower the seat. The natural position of this piston is extended, so it tends to elongate when the pressure exercised by the hairdresser is less than the force of the piston, so that when the hairdresser wants to raise his or her position, the piston's force will help this movement, lightening the muscular effort made without the action of the piston. To match the piston's force to the hairdresser's weight, versions in which the piston is adjustable are envisaged. The backrest can be slightly tilted and has conventional means for activating the piston valve such as an activation system consisting of a lever system like that used in an office chair with lift cylinder. So, in relation to what has been described, when the hairdresser wishes to lower his or her position, he will lean on the backrest which will tilt, activating the piston valve and enabling the seat and backrest to descend. It must be stressed that by acting on the piston drive in this way, the hairdresser does not use his or her hands, enabling him to work unhampered by operations which would complicate it or slow him down.

The seat and backrest are integrated in a frame which is preferably tubular. This frame has a side elevation with quasi-orthogonal geometry, where in the vertical part the hairdresser's seat and backrest are fixed and in the horizontal part, parallel to the floor, is the point of anchorage and turning about the client's chair. The length of the horizontal part can be adjusted to suit the hairdresser's work position using known methods such as a system of concentric telescopic rods or the sliding of a tubular system using linear bearings. The free far end of the horizontal part of the frame will be fixed to a turning element surrounding the base of the client's chair, so enabling the frame, and so the worker's station, to enable the hairdresser to work comfortably in his or her natural workspace. The hairdresser will turn the structure about the client's chair manually, no additional mechanical action being necessary.

The horizontal part of the frame has at least one rolling element in contact with the floor giving support to the assembly and making it sturdier and more stable.

### Description of the Drawings

For ease of understanding of everything described in this report, drawings are included in which a list of figures of the proposed invention are represented by way of example.

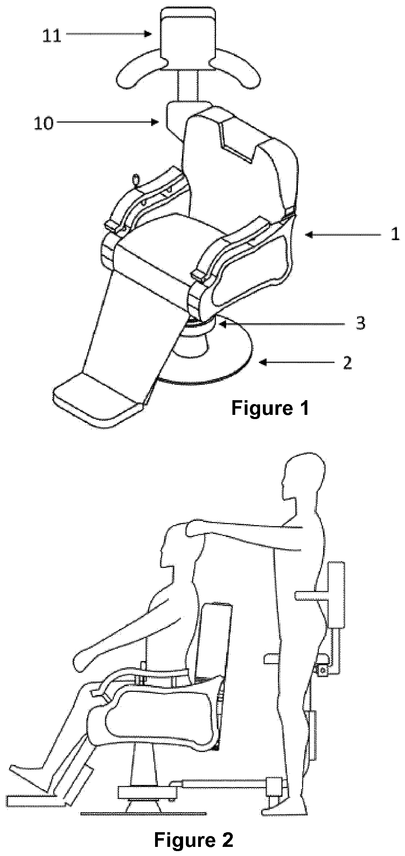

**Figure 1** shows an isometric view of the workstation in which the following can be seen:
   1. Customer's chair
   2. Base of the customer's chair
   3. Turning element
   10. Seat
   11. Backrest
**Figure 2** shows a side elevation of the workstation at its position of maximum height, with the hairdresser standing.

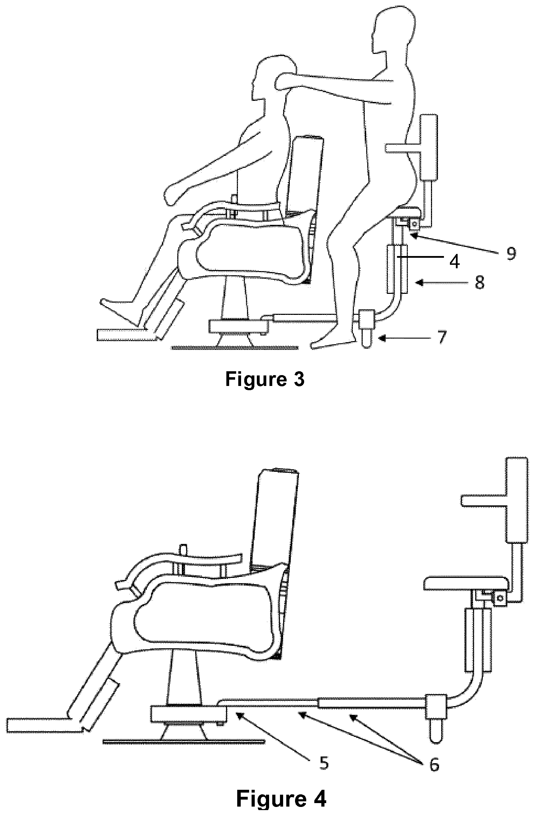

**Figure 3** shows a side elevation of the workstation in which the hairdresser is in a position with his legs half bent, in which the following can be seen:
   4. Frame
   7. Rolling support
   8. Lift system
   9. Lift system activation system
**Figure 4** shows a side elevation of the workstation with the telescopic tubular structure (**6**) extended, where the anchorage (**5**) can also be seen. The workstation has moved away from the client's chair with the seat/backrest assembly at its lowest position.

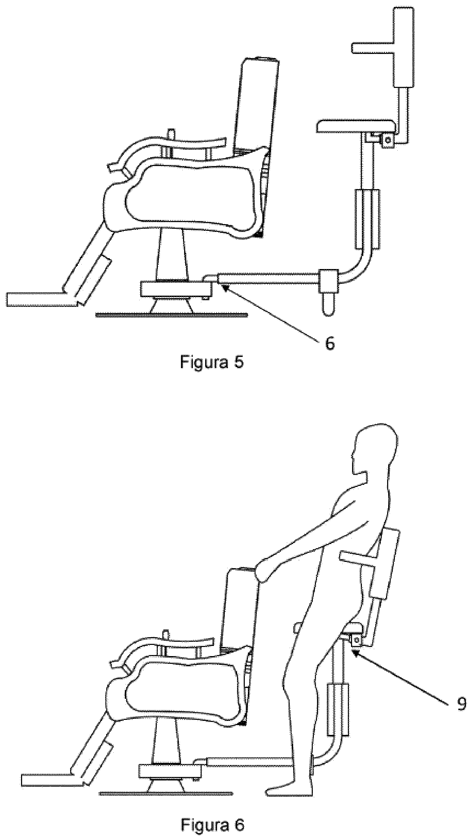

**Figure 5** shows a side elevation of the workstation with the telescopic tubular structure (**6**) withdrawn and the seat/backrest assembly at its highest position.
**Figure 6** shows a side elevation of the workstation in which the hairdresser is leaning against the backrest activating the lift system's actuation system (9).

### Description of a Preferential Embodiment

According to the invention the new ergonomic workstation with assisted movement for hairdressers is proposed with a saddle-type seat (**10**) and supporting backrest with armrests (**11**) joined to a vertical section of a frame (**4**), a side elevation of which has a right-angle and the horizontal section of the frame (**4**) parallel to the floor of which is fixed at its free far end to a turning element (**3**) with an anchorage (**5**) which has a torque between the two elements. The turning element surrounds the base (**2**) of a client's chair (**1**) and is fixed to it by a screwed clamp joint.

The length of the horizontal section of the frame to be adjustable to the hairdresser's work position by the use of a telescopic tubular structure (**6)** with concentric rods.

The height of the seat/backrest assembly is adjusted with a conventional lift system (**8**) of a gas piston, located in the vertical section of the frame under the seat, which is in its natural extended position when its valve is not operated and so tends to elongate when the pressure exercised by the hairdresser is less than the force of the piston. When the hairdresser wants to lower his position, he activates the gas piston valve by making a movement to tilt the backrest, which orders activation of the valve through an activation system (**9**) consisting of a set of levers which join the backrest to the piston actuation valve, producing the consequent descent of the seat/backrest assembly.

It has at least one rolling support (**7**) as a caster wheel located in the horizontal section of the frame.

## Claims

1. An ergonomic workstation with assisted movement for hairdressers enabling adjustment of the hairdressing worker's workspace, both in its vertical dimension and in his horizontal reach, enabling the hairdresser to work sitting down, with bent knees or standing up, wherein the ergonomic workstation comprises a saddle-type seat (**10**) and a supporting backrest with armrests (**11**), both joined to a vertical section of a frame (**4**), a side elevation of which has a right angle and the horizontal section of the frame (4) parallel to the floor of which is fixed at its free far end to a turning element (**3**) with an anchoring element (**5**) with a torque between them; the turning element surrounds the base (**2**) of a client's chair (**1**) and is fixed by a screwed clamp joint; wherein the length of the horizontal section of the frame (**4**) is changeable according to the hairdresser's requirements by the use of a telescopic tubular structure (**6**) of concentric rods; the height of the seat (**10**) and backrest (**11**) is adjusted by a lift system (**8**) of a gas piston located in the vertical section of the frame (**4**) under the seat (**10**), which is in its natural extended position when its valve is not operated, the valve being activated when the hairdresser makes a slight movement to tilt the backrest (**11**), use of his hands therefore being unnecessary, this action ordering activation of the gas piston and the consequent descent of the seat/backrest (**10,11**) assembly through an activation system (**9**) consisting of a set of levers; it has at least one caster wheel as a rolling support (**7**) located in the horizontal section of the frame (**4**).

## Patentansprüche

1. Neuer ergonomischer arbeitsplatz mit unterstützter bewegung für Friseure, sowohl in seiner vertikalen Dimension als auch in seiner horizontalen Reichweite angepasst werden kann, sodass der Friseur im Sitzen, mit gebeugten Knien oder im Stehen arbeiten kann **dadurch gekennzeichnet** hat es einen Sattelsitz (**10**) und eine tragende Rückenlehne mit Armlehnen (**11**), die beide mit einem vertikalen Rahmens (**4**) verbunden sind, dessen Seitenansicht, die einen nahezu rechten Winkel umfasst horizontaler Teil parallel zu dessen Boden für an einem Drehelement (**3**) mit einem Verankerungselement (**5**) mit einem Drehmoment zwischen ihnen befestigt ist; Das Drehelement umgibt die Basis (**2**) des Kundenstuhls (**1**) und wird mit einer Schraubklemme befestigt. Die einstellbar Länge des horizontalen Abschnitts des Rahmens entspricht den Anforderungen von des Friseurs durch die Verwendung einer teleskopischen röhrenförmigen Struktur (**6**) aus konzentrischen Stäben geändert werden. Die Höhe des Sitzes und der Rückenlehne wird durch ein Hebesystem (**8**) eingestellt durch einen Gaskolben, der sich im vertikalen des Rahmens unter dem Sitz befindet und sich in seiner natürlichen ausgefahrenen Position befindet, wenn sein Ventil nicht betätigt wird, letzteres Wird aktiviert, wenn der Friseur eine leichte Bewegung ausführt, um die Rückenlehne zu kippen. Die Verwendung seiner Hände ist daher nicht erforderlich. Diese Aktion ordnet die Aktivierung des Kolbens und das anschließende Absenken der Sitz-/ Rückenlehnenanordnung durch ein Aktivierungssystem (**9**) an, das aus einem Satz besteht von Hebeln; es hat mindestens ein Lenkrad als Rollstütze (**7**) im horizontalen Abschnitt des Rahmens (**4**).

## Revendications

1. Nouveau poste de travail ergonomique avec une assistance au movement pour des coiffeurs, à la fois dans sa dimension verticale et dans sa portée horizontale, permettant au coiffeur de travailler assis, les genoux pliés ou debout **caractérisé par** une siège de type selle (**10**) et un dossier de support avec accoudoirs (**11**), tous deux reliés en verticale d'un cadre (**4**) dont l'élévation latérale a un angle droit et une partie horizontale parallèle au plancher qui est fixée pour un élément tournant (**3**) avec un élément d'ancrage (**5**) avec un couple entre eux; l'élément tournant entoure la base (**2**) de la chaise du client (**1**) et est fixé par un joint de serrage vissé; la longueur de la section horizontale du cadre reglable selon les exigences du coiffeur par l'utilisation d'une structure tubulaire télescopique (**6**) de tiges concentriques; la hauteur du siège et du dossier est réglée par un système de levage (**8**) pour un piston à gaz situé dans la verticale du cadre sous le siège, qui est dans sa position d'extension naturelle lorsque sa valve n'est pas actionnée, ce dernier étant activé lorsque le coiffeur fait un léger mouvement pour incliner le dossier, l'utilisation de ses mains étant donc inutile, cette action ordonnant l'activation du piston et la descente conséquente de l'ensemble siège/dossier à travers un système d'activation (**9**) constitué d'un ensemble de leviers; il présente au moins une roue pivotante comme support roulant (**7**) située dans la section horizontale du châssis (**4**).
